# EUROPÄISCHE PATENTANMELDUNG

(11) **EP 3 132 833 A1**
(43) Veröffentlichungstag der Anmeldung: **22.02.2017**
(21) Anmeldenummer: 16173545.1
(22) Anmeldetag: 08.06.2016
(51) Int. Cl.: A63B 69/00, G02C 7/16, A61F 9/04, A63B 102/16

(54) **VORRICHTUNG ZUR BEGRENZUNG DES SICHTFELDES EINES MENSCHEN**

(30) Priorität: 12.08.2015 EP 15401086
(71) Anmelder: Tuncer, Abbdurrahman, 68333 Völklingen (DE)
(72) Erfinder: Tuncer, Abbdurrahman, 68333 Völklingen (DE)
(74) Vertreter: Sartorius, Peter

(57) **Zusammenfassung**

Die Erfindung betrifft eine Vorrichtung zur Begrenzung des Sichtfeldes eines Menschen mit Hilfe eines am Körper oder am Kopf zu befestigenden Tragteils.

Der Erfindung liegt die Aufgabe zugrunde, die Vorrichtung zur Begrenzung des Sichtfeldes eines Menschen auf einfache und kostengünstige Weise herzustellen und das Sichtfeld so zu begrenzen, dass die Person beim sportlichen Einsatz ihren Blick hauptsächlich auf sein Gegenüber richtet und mit Hilfe der Vorrichtung daran gehindert wird, auf ihre Füße zu schauen.

Gelöst wird die Aufgabe erfindungsgemäß dadurch, dass das Tragteil (2) eine Einrichtung aufweist, mit deren Hilfe das Sichtfeld des Menschen auf einen Ausschnitt eingeschränkt wird.

## Beschreibung

Vorrichtung (1) zur Begrenzung des Sichtfeldes eines Menschen mit Hilfe eines am Körper oder am Kopf zu befestigenden Tragteils (2) wobei das einstellbare Tragteil (2) eine Einrichtung aufweist, mit deren Hilfe das Sichtfeld des Menschen auf einen Ausschnitt eingeschränkt wird wobei dass das oder ein in etwa flaches Teil (10, 11) als Band (3) ausgebildet ist, das elastisch und/oder längenveränderlich ausgebildet ist und am Kopf des Menschen befestigt werden kann wobei das Tragteil (2) aus einem festen Material besteht, das an Teilen des Kopfes oder an der Nase und/oder an den Ohren (13) abstützbar ist und dass das Tragteil aus einem oder mehreren Teilen (3, 4, 5, 6) besteht, die fest und/oder einteilig miteinander verbunden sind, um es unterschiedlichen Kopfformen anzupassen, , dass sie das Sichtfeld des Menschen zumindest zu einer Seite oder in eine Richtung begrenzen, wobei zumindest ein oder mehrere Teile des Tragteils (2) unterhalb der Augen am Kopf des Menschen befestigt werden

Es ist aus der DE 94 04 439U1 Neuerung bekannt, die eine Einrichtung zur Begrenzung des Blickfeldes abwärts einer im wesentlichen horizontalen Blickebene betrifft, Die Einrichtung für einen Fußballspieler vorgesehen, damit er während des Fußballtrainings seinem einer den Kopf und seinen Augen auf den angreifenden Spielers ausgerichtet sein soll. Die Einrichtung umfasst eine Befestigungs- und Trageeinheit, die mit einem Gestell verbunden ist, das einen auf die Nase des Spielers aufsetzbaren Bügel aufweist. Allgemein und insbesondere aus dem Bereich des Sports sind Vorrichtungen bekannt, bei denen Gläser bzw. durchsichtige Sichtblenden an einem Stirnband, einem elastischen Gurtband oder Bügeln befestigt sind. Stirnband, Gurtband, Bügel und Augengläser bzw. Sichtblenden bilden dabei eine fest verbundene Einheit. Aus der DE-PS 871 948 ist eine Schutzvorrichtung für Fahrzeugführer gegen Blendwirkungen von entgegenkommenden Fahrzeugen bekannt, die übliche Brillengläser bzw. ein einstückiges Sichtglas aufweist, die an üblichen Brillenbügeln oder einem elastischen Halteband befestigt sind. An zumindest einem der Brillengläser ist eine undurchsichtige Sichtblende angeordnet, die vom Träger bei Auftritt von Blendwirkungen eines entgegenkommenden Fahrzeuges um einen gewünschten Winkel seitlich verschwenkt werden kann und nach Wegfall der Blendwirkung wieder in die Ausgangsstellung zurückgeklappt wird. Diese Abblendvorrichtung hat den Nachteil, dass die Sicht nach unten nicht versperrt werden kann. Aus der DE-PS 285971 ist gleichfalls eine Schutzbrille für Fahrzeugführer bekannt, die insbesondere die Augen gegen Witterungseinflüsse, wie Regentropfen, Schnee, Hagel usw. schützen soll. Zu diesem Zweck sind am Brillengestell oben und unten um jeweils horizontale Achsen schwenkbare obere und untere Glashälften vorgesehen, die nach außen oder innen weggeklappt werden können, um den Blickwinkel für den Träger zu verringern oder zu vergrößern. Der Blickwinkel wird jedoch durch einen Bereich gebildet, der jeweils außerhalb der oberen und unteren Glashälfte liegt. Da die untere Glashälfte durchsichtig ist, wird die Sicht nach unten nicht versperrt. Spezielle Einrichtungen, Brillen oder Masken, die insbesondere für spezifische Trainingszwecke im Fußballsport geeignet sind, sind nicht bekannt. Es ist daher Aufgabe der bekannten Neuerung, eine Einrichtung vorzugsweise für Fußballspieler zu schaffen, mit der das Training am Ball derart optimiert wird, dass während des Trainings lediglich die Spielumgebung, nicht jedoch der Fußbereich des Spielers beobachtet werden kann. Neuerungsgemäß wird die Aufgabe dadurch gelöst, dass das Gestell im Wesentlichen horizontal von den Augen des Spielers weggerichtete, zumindest nahezu undurchsichtige Flächengebilde aufweist. Die Flächengebilde weisen dabei eine Tiefe auf, die die Sicht auf den Fußbereich des Spielers verdecken. Dazu bestehen die Flächengebilde aus einem nahezu undurchsichtigen bzw. einem vollständig undurchsichtigen Kunststoffmaterial. In bevorzugter Ausführung der Neuerung sind die Flächengebilde und der auf die Nase des Spielers aufsetzbare Bügel einstückig ausgebildet. Des Weiteren kann das Gestell zumindest teilweise gepolstert sein. Die Befestigungs- und Trageeinheit bildet mit dem Gestell in bevorzugter Ausführungsform der Neuerung einen geschlossenen Ring. Ebenfalls bevorzugt besteht die Befestigungs- und Trageeinheit aus einem elastischen Gurtband, das in seiner Länge verstellbar sein kann.

Der Erfindung liegt die Aufgabe zugrunde, die Vorrichtung zur Begrenzung des Sichtfeldes eines Menschen auf einfache und kostengünstige Weise herzustellen und das Sichtfeld so zu begrenzen und hierzu so optimal anzuordnen und/oder zu verändern, dass die Person beim sportlichen Einsatz ihren Blick hauptsächlich auf sein Gegenüber richtet und mit Hilfe der Vorrichtung daran gehindert wird, auf ihre Füße zu schauen.

Die Aufgabe wird erfindungsgemäß durch folgende Merkmale gelöst,
a) Vorrichtung (1) zur Begrenzung des Sichtfeldes an dem Tragteil (2) in einem Winkel α angeordnet ist, eine Größe zwischen 50° und 70° oder zwischen 55° und 65° oder zwischen 58° und 62° aufweist,
b) der Winkel α ist mit Hilfe einer Einstellvorrichtung in einem Größenbereich zwischen 50° und 70° veränderbar,
c) die Einstellvorrichtung ist als Gelenkverbindung (17) ausgebildet;
d) die Gelenkvorrichtung ist mit Hilfe einer Klemmvorrichtung und oder Kupplungseinrichtung in Form einer Form-und/oder Kraftschlussverbindung ausgestattet.

Durch die vorteilehafte Positionierung der Vorrichtung (1) zur Begrenzung des Sichtfeldes eines Menschen mit Hilfe eines am Körper oder am Kopf zu befestigenden Tragteils (2) in einer ganz bestimmten Winkellage wird sichergestellt, dass der Fußballspieler bei Tragen der Vorrichtung ein ausreichend Gesichtsfeld bleibt und er durch Einsatz dieser Vorrichtung beim Fußballspiel nicht beeinträchtigt wird. Abweichungen von der optimalen Positionierung der Vorrichtung bzw. der optimalen Winkeleinstellung von 60° bis 70°stellen eine äußerst starke Behinderung oder auch Gefährdung für den Fußballspieler dar, was man bisher einfach nicht erkannt hat, sodass die bisher bekannten Vorrichtungen auch nicht zum Einsatz gekommen sind. Hierdurch wird erstmalig auf einfache und kostengünstige Weise sichergestellt, dass die Person oder der Fußballspieler während des Fußballspiels seinen Blick nicht auf seine Füße ausrichtet, sondern auf den Fußballgegner und dadurch sämtliche Aktionen in kürzester Zeit wahrnimmt und darauf mit der richtigen Maßnahme reagiert. Würde der Fußballspieler, wie bisher üblich, seinen Blick nach unten ausrichten und unter anderem auf seine Füße blicken und erst danach den Gegner ins Visier nehmen, verlöre er wertvolle Zeit, die er benötigt, alle Aktionen des Gegner wahrzunehmen. Der Blickwinkelwechsel von unten nach oben und vorne in Richtung des Gegners benötigt zwar nur eine sehr kurze Zeit, eventuell Millisekunden, die aber bei einem schnellen Kampfsport von großer Bedeutung sein können. Es ist dabei zu beachten, dass bei einem Kampfsport viele Aktionen im Millisekunden-Bereich ausgeführt werden. Diese Aktionen müssen aber von der angreifenden Person oder dem Fußballspieler erfasst, verarbeitet und mit der richtigen Aktion beantwortet werden. Betrachtet man heute die Fußballspieler im Einsatz, so kann man sehr häufig beobachten, dass sie während des Fußballspiels ihre Blicke nicht auf den Gegner ausrichten, sondern vielfach auf den Bereich ihrer Füße. Hinweise des Trainers, der Spieler sollen nur den Gegner im Auge haben, helfen wenig, da während des Spiels solche Ermahnungen leicht in Vergessenheit geraten. Hier will also die Erfindung Abhilfe schaffen und dafür sorgen, dass der agierende Spieler stets seine Blicke in Richtung des Gegners ausrichtet. Schaut er dennoch nach unten, wird er feststellen, dass er dort nicht sieht, sodass er nach einer gewissen Gewöhnungszeit lernt, nicht nach unten sondern nach vorne in Richtung des Gegners zu schauen. Durch Verwendung der Gelenkverbindung, die auch einstellbar ausgebildet sein kann, kann die optimale Winkellage des flachen Teils der Vorrichtung gewählt werden. Da die Gelenkverbindung mit Hilfe einer Klemmvorrichtung und oder Kupplungseinrichtung in Form einer Form-und/oder Kraftschlussverbindung ausgestattet ist, kann je nach Wunsch sehr leicht eine Korrektur der Winkellage des flachen Teils auch währende des Spiels vorgenommen werden und nach der Einstellung sicher gestellt werden, dass nach der optimalen Positionierung während des Spiels des Fußballspielers keine ungewünschte weitere Verstellung des flachen Teils erfolgt. Hierdurch wird also eine Vorrichtung geschaffen, die genau auf die Anforderungen eines Spieles einstellbar ist. Die bekannten Vorrichtungen waren den hier beschriebenen Anforderungen nicht gewachsen Hierzu ist es vorteilhaft, dass das Tragteil als Band ausgebildet ist, das elastisch und/oder längenveränderlich ausgebildet ist und am Kopf des Menschen befestigt werden kann. Mit Hilfe des elastisch und/oder längenveränderlich ausgebildeten Tragteils lässt sich dieses in kürzester Zeit am Kopf in die gewünschte, richtige Position verstellen und festsetzen, sodass es beim Spiel seine Position beibehält. Sollte es dennoch verrutschen, kann die Spannung des Bandes verändert werden und das Tragteil ohne weiteres wieder in die richtige Position zurückverstellt und langhaltig besser gesichert werden.

Eine zusätzliche Möglichkeit ist gemäß einer Weiterbildung der Erfindung, dass das Tragteil aus einem festen Material besteht, das an Teilen des Kopfes oder an der Nase und/oder an den Ohren abstützbar ist, wobei das Tragteil aus einem oder mehreren Teilen besteht, die fest und/oder einteilig miteinander verbunden sind und an unterschiedliche Kopfformen angepasst werden können.

Ferner ist es vorteilhaft, dass zumindest ein Teil oder das vordere Teil ein sich auf dem Nasenrücken abstützendes Formteil aufweist, an das sich an beiden Seitenenden des Formteils je ein weiteres oder ein in etwa flaches Teil anschließt, sodass das Sichtfeld des Menschen nach unten auf einen bestimmten Bereich begrenzt ist oder so begrenzt werden kann, dass ein kleiner Bereich der Standfläche des Menschen nicht wahr genommen werden kann.

Vorteilhaft ist es auch, dass an beiden Seitenenden des Abstützteils je ein in etwa flaches Teil anschließt, das mit Bezug auf die Standfläche des Menschen horizontal verläuft oder zumindest horizontal einstellbar ist. Die Verstellmöglichkeit des flachen Teils stellt sicher, dass es immer so ausgerichtet werden kann, dass ein kleiner Bereich der Standfläche des Menschen nicht wahrgenommen werden kann.

Vorteilhaft ist es, dass das flache und/oder horizontal einstellbare Teil, das beiderseits des abstützbaren Teils und/oder des Formteils vorgesehen ist, mit dem abstützbaren Teil und/oder Formteil beweglich und/oder einstellbar und/oder lösbar verbunden ist.

Von besonderer Bedeutung ist für die vorliegende Erfindung, dass das flache und/oder horizontal einstellbare Teil, das beiderseits des abstützbaren Teils und/oder Formteils vorgesehen ist, mit je einem weiteren seitlich am Kopf verlaufenden Teil beweglich und/oder einstellbar und/oder lösbar verbunden ist. Auf diese Weise erhält man eine sehr gute Anpassung an den Kopfumfang des Spielers.

Auch ist es vorteilhaft, dass sich das seitlich am Kopf verlaufende Teil an eine seitliche Kopffläche anlegt und/oder auf den Ohren des Kopfes abgestützt werden kann.

Ferner ist es vorteilhaft, dass die vorderen Teile des Sichtfeldes eines Menschen verstellbar ausgebildet und/oder miteinander einstellbar verbunden sind, wobei der Querschnitt zumindest einzelner Teile flach, oval oder so ausgebildet sind, dass sie das Sichtfeld des Menschen zumindest zu einer Seite oder in eine Richtung begrenzen, wobei zumindest ein oder mehrere Teile des Tragteils unterhalb der Augen am Kopf des Menschen befestigt werden.

Vorteilhaft ist es auch, dass das am Körper oder am Kopf zu befestigende Tragteil die Kopfform ganz umschließt und der hintere Teil mit Bezug auf den Kopf eines Menschen als einstellbares Teil und/oder elastisch einstellbares und/oder längenveränderliches Teil oder Band ausgebildet ist.

Ferner ist es vorteilhaft, dass einzelne oder alle Teile des am Kopf zu befestigen Tragteils Einzelelemente sind, die mit dem jeweils benachbarten Teil fest und/oder lösbar und/oder beweglich und/oder einstellbar verbunden sind und dass das Tragteil ein weiteres Befestigungsteil aufweist, mit dessen Hilfe das Tragteil noch sicherer am Kopf befestigt werden kann.

Weitere Vorteile und Einzelheiten der Erfindung sind in den Patentansprüchen und in der Beschreibung erläutert und in den Figuren dargestellt.

Dabei zeigen:
- Fig. 1: eine perspektivische Darstellung der Vorrichtung zur Begrenzung des Sichtfeldes eines Menschen mit Hilfe eines Tragteils, das als Materialbogen unterschiedlicher Breite am Kopf eines Menschen befestigt werden kann;
- Fig. 2: eine Draufsicht, gemäß Fig. 1;

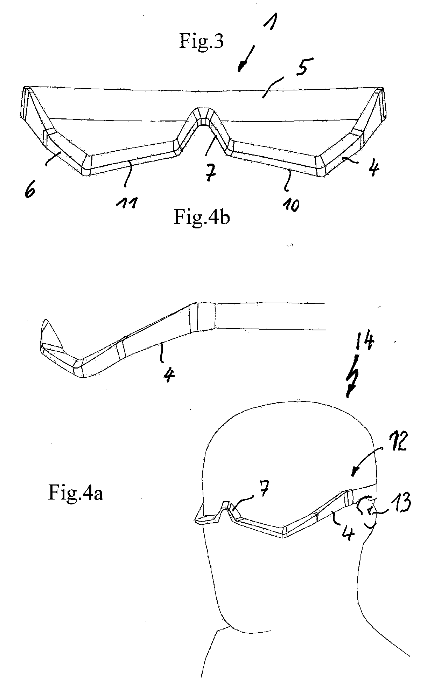

- Fig. 3: eine perspektivische Vorderansicht, gemäß Fig. 1;
- Fig. 4a: das auf dem Kopf eines Athleten oder Fußballspielers aufgesetzte Tragteil;
- Fig. 4b: einen seitlichen Bügel des Tragteils;

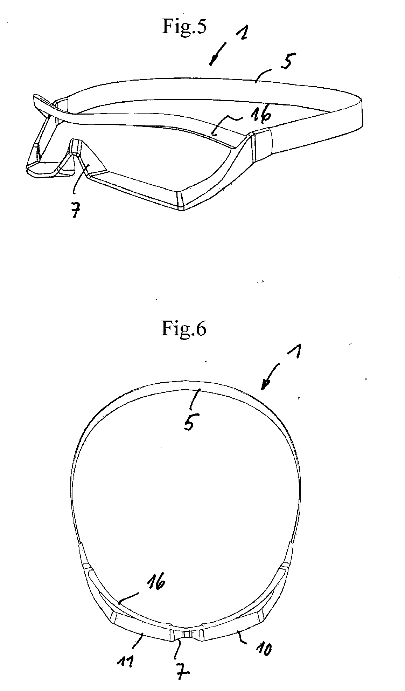

- Fig. 5: ein weiteres Ausführungsbeispiel des Tragteils mit einem oberhalb der Augen vorgesehenen Halteband;
- Fig. 6: eine Draufsicht, gemäß Fig. 5;

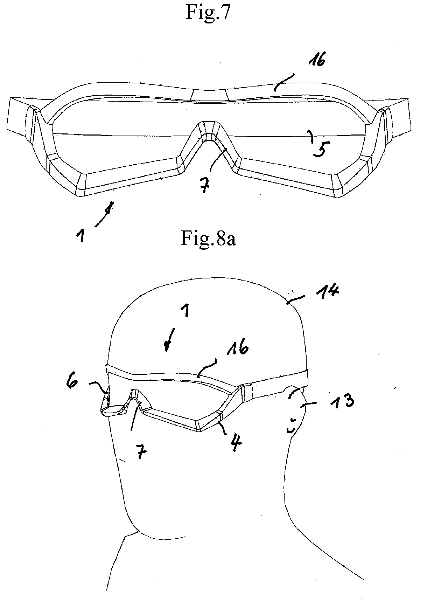

- Fig. 7: eine perspektivische Darstellung in der Ansicht von vorne des Ausführungsbeispiels des Tragteils, gemäß Fig. 5 mit einem oberhalb der Augen vorgesehenen Halteband;
- Fig. 8a: ein auf dem Kopf eines Akteurs aufgesetzte Tragteil, gemäß Fig. 5;

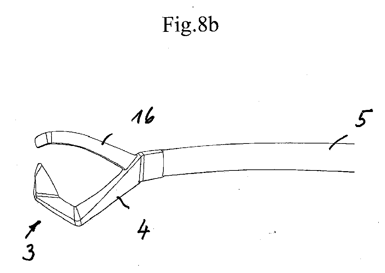

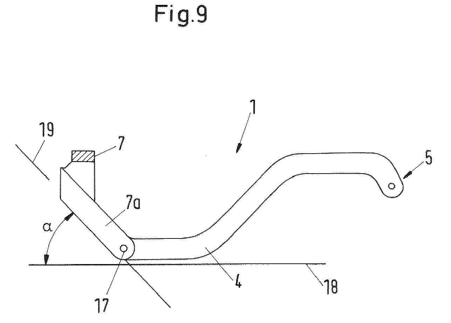

- Fig. 8b: die Schräglage bzw. Winkellage des veränderbaren Formteils In Seitenansicht gemäß Fig. 5.

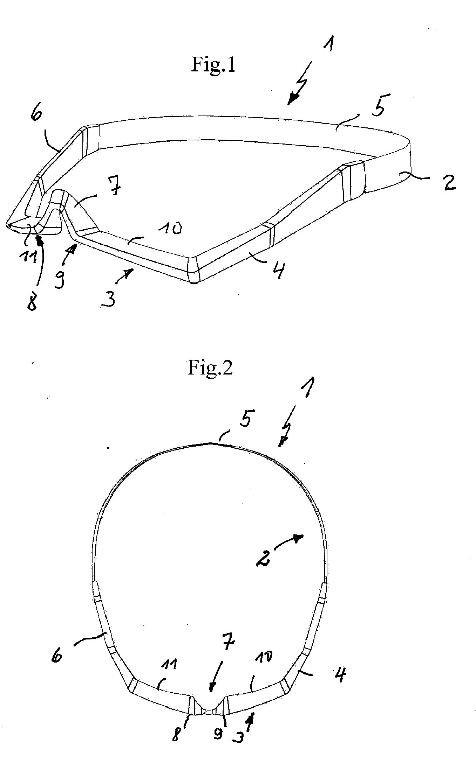

In Fig. 1 ist eine Vorrichtung mit 1 bezeichnet, die eine Einrichtung aufweist, mit deren Hilfe das Sichtfeld des Menschen auf einen Ausschnitt eingeschränkt wird. Der hier erwähnte Mensch kann ein Athlet sein, der gegen einen anderen Athleten im Kampfsport, wie Boxen oder Fußballspiel, antritt.

Hierdurch wird auf einfache Weise sichergestellt, dass die Person z. B. während des Fußballspiels ihren Blick nicht auf ihre Füße ausrichtet, sondern auf den Gegner und dadurch sämtliche Aktionen in kürzester Zeit wahrnimmt und darauf mit der richtigen Maßnahme reagiert. Würde der Fußballspieler, wie oft üblich, seinen Blick nach unten ausrichten und unter anderem nur auf seine Füße blicken und erst danach den Gegner ins Visier nehmen, verlöre er wertvolle Zeit, die er benötigt alle Aktionen des Gegner rechtzeitig wahrzunehmen. Der Blickwechsel von unten nach oben und wieder nach vorne in Richtung des Gegners benötigt zwar nur eine sehr kurze Zeit, eventuell Millisekunden, die aber bei einem schnellen Kampfsport von sehr großer Bedeutung sein können. Es ist dabei zu beachten, dass bei einem Kampfsport viele Aktionen in Millisekunden Bereich ausgeführt werden. Diese Aktion müssen aber von der angreifenden Person oder des Fußballspielers sofort erfasst, verarbeitet und mit der richtigen Aktion beantwortet werden.

Betrachtet man die Fußballspieler im Einsatz, so kann man häufig beobachten, dass sie während des Fußballspiels ihre Blicke nicht auf den Gegner ausrichten, sondern vielfach auf den Bereich ihrer Füße. Hinweise des Trainers, der Spieler soll nur den Gegner im Auge haben, helfen wenig, da während des Spiels solche Ermahnungen leicht in Vergessenheit geraten. Hier will also die Erfindung Abhilfe schaffen und dafür sorgen, dass der Spieler stets seine Blicke in Richtung des Gegners ausrichtet. Schaut er dennoch nach unten, wird er feststellen, dass er dort nicht sieht, sodass er nach einer gewissen Gewöhnungszeit lernt, nicht nach unten sondern nur nach vorne in Richtung des Gegners zu schauen. Die Sehgewohnheiten lassen sich mit Hilfe eines im Tragteil vorgesehenen Sensors erfassen und an einen Rechner übertragen, um auf diese Weise die Sehgewohnheiten des Spielers zu ermitteln und nach und nach zu korrigieren. Deshalb ist es vorteilhaft, wenn das Tragteil mit der Sehbegrenzung insbesondere zu Trainingszwecken eingesetzt wird.

Hierzu ist das Tragteil 2 als Band 3 ausgebildet, das elastisch und/oder längenveränderlich ausgebildet ist und am Kopf des Menschen befestigt werden kann. Mit Hilfe des elastisch und/oder längenveränderlich ausgebildeten Tragteils 2 lässt sich dieses in kürzester Zeit am Kopf 14 in die gewünschte richtige Position verstellen und festsetzen, sodass es beim Spiel seine Position beibehält. Sollte es dennoch verrutschen, so kann die Spannung des Bandes verändert werden und das Tragteil 2 ohne weiteres wieder in die richtige Position zurückverstellt und langhaltig gesichert werden.

Eine zusätzliche Möglichkeit besteht darin, dass das Tragteil 2 aus einem festen Material besteht, das an Teilen des Kopfes 14 oder an der Nase und/oder an den Ohren 13 abstützbar ist, wobei das Tragteil 2 aus einem oder mehreren Teilen 3, 4, 5, 6 bestehen kann, die fest und/oder einteilig miteinander verbunden sind, um es unterschiedlichen Kopfformen anzupassen. Zwischen dem Sichtschutz 7a und dem Teil 4 kann in vorteilhafterweise eine Feder oder Spannvorrichtung vorgesehen sein, die so eingestellt ist und dafür sorgt, dass das der Sichtschutz 7a und/oder die zugehörigen Teile 7 fest gegen die Stirn und/oder den Nasenrücken des Spielers gedrückt werden.

Ferner ist es vorteilhaft, dass zumindest ein Teil oder das vordere Teil 3 ein sich auf dem Nasenrücken abstützendes Formteil oder die Sicht des Spielers zumindest teilweise verdeckenden Sichtschutzes 7 aufweist, an das sich an beiden Seitenenden 8, 9 des Formteils 7 je ein weiteres oder ein in etwa flaches Teil 10, 11 anschließt, sodass das Sichtfeld des Menschen nach unten auf einen bestimmten Bereich begrenzt ist oder so begrenzt werden kann, dass ein kleiner Bereich der Standfläche des Menschen nicht wahr genommen werden kann. Das in etwa flache Teil kann jede beliebige Querschnittsform aufweisen, die aber so ausgebildet sein sollte, dass das Gesichtsfeld auf jeden Fall ausreichend begrenzt wird. Die Verbindungselemente zwischen den einzelnen Teilen können elastische Verbindungselemente oder kleine Scharniere sein, die auch mit Federelementen ausgestattet werden können, um sicherzustellen, dass die einzelnen Teile an die Kopfform angedrückt werden.

Vorteilhaft ist es auch, dass an beiden Seitenenden 8, 9 des Abstützteils 7 je ein in etwa flaches Teil 10, 11 anschließt, das mit Bezug auf die Standfläche des Menschen horizontal verläuft oder zumindest horizontal oder in etwa horizontal einstellbar ist. Die Verstellmöglichkeit des flachen Teils stellt sicher, dass es immer so ausgerichtet werden kann, dass ein kleiner Bereich der Standfläche des Menschen nicht wahrgenommen werden kann.

Ferner ist es möglich, dassdas flache und/oder horizontal einstellbare Teil 10, 11, das beiderseits des abstützbaren Teils und/oder des Formteils 7 vorgesehen ist, mit dem abstützbaren Teil und/oder Formteil 7 beweglich und/oder einstellbar und/oder lösbar verbunden ist.

Von besonderer Bedeutung ist es auch, dass das flache und/oder horizontal einstellbare Teil 10, 11, das beiderseits des abstützbaren Teils und/oder Formteils 7 vorgesehen ist, mit je einem weiteren seitlich am Kopf verlaufenden Teil 4, 6 beweglich und/oder einstellbar und/oder lösbar verbunden ist. Auf diese Weise erhält man eine sehr gute Anpassung an den Kopfumfang des Spielers, da sich das seitlich am Kopf in etwa vertikal verlaufende Teil an eine seitliche Kopffläche anlegt und/oder auf den Ohren des Kopfes abgestützt werden kann.

Ferner ist es vorteilhaft, dass die vorderen Teile 3-11 des Sichtfeldes eines Menschen verstellbar ausgebildet und/oder miteinander einstellbar verbunden sind, wobei der Querschnitt zumindest einzelner Teile flach, oval oder so ausgebildet sind, dass sie das Sichtfeld des Menschen zumindest zu einer Seite oder in eine Richtung begrenzen, wobei zumindest ein oder mehrere Teile des Tragteils 2 unterhalb der Augen am Kopf des Menschen befestigt werden.

Vorteilhaft ist es auch, dass das am Körper oder am Kopf zu befestigende Tragteil 2 die Kopfform ganz umschließt und der hintere Teil mit Bezug auf den Kopf eines Menschen als einstellbares Teil und/oder elastisch einstellbares und/oder längenveränderliches Teil oder Band ausgebildet ist.

Auch ist es von Vorteil, dass einzelne oder alle Teile des am Kopf zu befestigen Tragteils Einzelelemente sind, die mit dem jeweils benachbarten Teil fest und/oder lösbar und/oder beweglich und/oder einstellbar verbunden sind. Zwischen dem Sichtschutz und dem Teil kann in vorteilhafterweise eine Feder oder Spannvorrichtung vorgesehen sein, die so eingestellt ist und dafür sorgt, dass das der Sichtschutz und/oder die zugehörigen Teile fest gegen die Stirn und/oder den Nasenrücken des Spielers gedrückt werden.

Damit ein noch besserer Halt des Tragteils 2 am Körper oder am Kopf 14 gewährleistet wird, weist das zu befestigende Tragteil 2 ein weiteres Befestigungsteil 16 auf, mit dessen Hilfe das Tragteil 2 noch sicherer am Kopf 14 befestigt werden kann. Das Befestigungsteil 16 ist hierzu an die Seitenteile 6 entweder fest oder beweglich angeschlossen.

Nach einem anderen Ausführungsbeispiel ist die Vorrichtung 1 zur Begrenzung des Sichtfeldes an dem Tragteil 2 in einem Winkel α angeordnet ist, eine Größe zwischen 50° und 70° oder zwischen 55° und 65° oder zwischen 58° und 62° aufweist.

Der Winkel α ist mit Hilfe einer Einstellvorrichtung in einem Größenbereich zwischen 50° und 70° mit Bezug auf die Standfläche 18 des Spieles veränderbar.

Die Einstellvorrichtung kann als Gelenkverbindung 17 mit einem in seiner Winkellage veränderbarem Formteil 7a ausgebildet, wobei die Gelenkvorrichtung mit Hilfe einer Klemmvorrichtung und oder Kupplungseinrichtung in Form einer Form-und/oder Kraftschlussverbindung ausgestattet ist. Hierzu ist das Formteil 7a über einen Gelenkbolzen an den Teil 4 der Vorrichtung 1 schwenkbar angeschlossen, wobei die Klemmvorrichtung und oder Kupplungseinrichtung in Form einer Form-und/oder Kraftschlussverbindung dem Gelenkbolzen zugeordnet ist. Zwischen dem Sichtschutz 7a und dem Teil 4 kann in vorteilhafterweise eine Feder oder Spannvorrichtung vorgesehen sein, die so eingestellt ist und da-für sorgt, dass das der Sichtschutz 7a und/oder die zugehörigen Teile 7 fest gegen die Stirn und/oder den Nasenrücken des Spielers gedrückt werden.

### Bezugszeichenliste

- **1**: Vorrichtung
- **2**: Tragteil
- **3**: Teil, Band
- **4**: Teil
- **5**: Teil
- **6**: Teil
- **7**: Formteil, Abstützteil
- 7a: in seiner Winkellage veränderbarer Formteil
- **8**: Seitenende
- **9**: Seitenende
- **10**: ein weiteres in etwa flaches Teil
- **11**: ein weiteres in etwa flaches Teil
- **12**: Kopffläche
- **13**: Ohr
- **14**: Kopf
- **16**: Befestigungsteil
- **17**: Gelenkverbindung
- 18: Standfläche des Spielers

## Patentansprüche

1. Vorrichtung (1) zur Begrenzung des Sichtfeldes eines Menschen mit Hilfe eines am Körper oder am Kopf zu befestigenden Tragteils (2) wobei das einstellbare Tragteil (2) eine Einrichtung aufweist, mit deren Hilfe das Sichtfeld des Menschen auf einen Ausschnitt eingeschränkt wird wobei dass das oder ein in etwa flaches Teil (10, 11) als Band (3) ausgebildet ist, das elastisch und/oder längenveränderlich ausgebildet ist und am Kopf des Menschen befestigt werden kann wobei das Tragteil (2) aus einem festen Material besteht, das an Teilen des Kopfes oder an der Nase und/oder an den Ohren (13) abstützbar ist und dass das Tragteil aus einem oder mehreren Teilen (3, 4, 5, 6) besteht, die fest und/oder einteilig miteinander verbunden sind, um es unterschiedlichen Kopfformen anzupassen, , dass sie das Sichtfeld des Menschen zumindest zu einer Seite oder in eine Richtung begrenzen, wobei zumindest ein oder mehrere Teile des Tragteils (2) unterhalb der Augen am Kopf des Menschen befestigt werden **gekennzeichnet durch folgende Merkmale,**
a) Vorrichtung (1) zur Begrenzung des Sichtfeldes an dem Tragteil (2) in einem Winkel α angeordnet ist, eine Größe zwischen 50° und 70° oder zwischen 55° und 65° oder zwischen 58° und 62° aufweist,
b) der Winkel α ist mit Hilfe einer Einstellvorrichtung in einem Größenbereich zwischen 50° und 70° veränderbar,
c) die Einstellvorrichtung ist als Gelenkverbindung (17) ausgebildet;
d) die Gelenkvorrichtung ist mit Hilfe einer Klemmvorrichtung und oder Kupplungseinrichtung in Form einer Form-und/oder Kraftschlussverbindung ausgestattet.

2. Vorrichtung nach einem der vorhergehenden Ansprüche,
**dadurch gekennzeichnet,**
zumindest ein Teil oder das vordere Teil (3) ein sich auf dem Nasenrücken abstützendes Formteil (7) aufweist, an das sich an beiden Seitenenden (8, 9) des Formteils (7) je ein weiteres oder ein in etwa flaches Teil (10, 11) anschließt, sodass das Sichtfeld des Menschen nach unten auf einen bestimmten Bereich begrenzt ist oder so begrenzt werden kann, dass ein kleiner Bereich der Standfläche des Menschen nicht wahr genommen werden kann.

3. Vorrichtung nach einem der vorhergehenden Ansprüche,
**dadurch gekennzeichnet,**
**dass** zumindest ein Teil oder das vordere Teil (3) ein sich auf dem Nasenrücken abstützendes Formteil (7) aufweist, an das sich an beiden Seitenenden (8, 9) des Formteils (7) je ein weiteres oder ein in etwa flaches Teil (10, 11) anschließt, sodass das Sichtfeld des Menschen nach unten auf einen bestimmten Bereich begrenzt ist oder so begrenzt werden kann, dass ein kleiner Bereich der Standfläche des Menschen nicht wahr genommen werden kann.

4. Vorrichtung nach einem der vorhergehenden Ansprüche,
**dadurch gekennzeichnet,**
**dass** an beiden Seitenenden (8, 9) des Abstützteils (7) je ein in etwa flaches Teil (10, 11) anschließt, das mit Bezug auf die Standfläche des Menschen horizontal verläuft oder zumindest horizontal einstellbar ist.

5. Vorrichtung nach einem der vorhergehenden Ansprüche,
**dadurch gekennzeichnet,**
**dass** das flache und/oder horizontal einstellbare Teil (10, 11), das beiderseits des abstützbaren Teils und/oder des Formteils (7) vorgesehen ist, mit dem abstützbaren Teil und/oder Formteil (7) beweglich und/oder einstellbar und/oder lösbar verbunden ist.

6. Vorrichtung nach einem der vorhergehenden Ansprüche,
**dadurch gekennzeichnet,**
**dass** das flache und/oder horizontal einstellbare Teil (10, 11), das beiderseits des abstützbaren Teils und/oder Formteils (7) vorgesehen ist, mit je einem weiteren seitlich am Kopf verlaufenden Teil (4, 6) beweglich und/oder einstellbar und/oder lösbar verbunden ist.

7. Vorrichtung nach einem der vorhergehenden Ansprüche,
**dadurch gekennzeichnet,**
**dass** sich das seitlich am Kopf verlaufende Teil (4, 6) an eine seitliche Kopffläche (12) anlegt und/oder auf den Ohren (13) des Kopfes abgestützt werden kann.

8. Vorrichtung nach einem der vorhergehenden Ansprüche,
**dadurch gekennzeichnet,**
**dass** die vorderen Teile (3 -11) des Sichtfeldes eines Menschen verstellbar ausgebildet und/oder miteinander einstellbar verbunden sind, wobei der Querschnitt zumindest einzelner Teile flach, oval oder so ausgebildet sind, dass sie das Sichtfeld des Menschen zumindest zu einer Seite oder in eine Richtung begrenzen, wobei zumindest ein oder mehrere Teile des Tragteils (2) unterhalb der Augen am Kopf des Menschen befestigt werden.

9. Vorrichtung nach einem der vorhergehenden Ansprüche,
**dadurch gekennzeichnet,**
**dass** das am Körper oder am Kopf zu befestigende Tragteil (2) die Kopfform ganz umschließt und der hintere Teil mit Bezug auf den Kopf eines Menschen als einstellbares Teil und/oder elastisch einstellbares und/oder längenveränderliches Teil oder Band ausgebildet ist.

10. Vorrichtung nach einem der vorhergehenden Ansprüche,
**dadurch gekennzeichnet,**
**dass** einzelne oder alle Teile des am Kopf zu befestigen Tragteils (2) Einzelelemente sind, die mit dem jeweils benachbarten Teil fest/oder lösbar und/oder beweglich und/oder einstellbar verbunden sind.

11. Vorrichtung nach einem der vorhergehenden Ansprüche,
**dadurch gekennzeichnet,**
**dass** das am Körper oder am Kopf (14) zu befestigende Tragteil (2) ein weiteres Befestigungsteil (16) aufweist, mit dessen Hilfe das Tragteil noch sicherer am Kopf befestigt werden kann, wobei zwischen dem Sichtschutz 7a und dem Teil 4 kann ist in vorteilhafterweise eine Feder oder Spannvorrichtung vorgesehen, die so eingestellt ist und dafür sorgt, dass das der Sichtschutz 7a und/oder die zugehörigen Teile 7 fest gegen die Stirn und/oder den Nasenrücken des Spielers gedrückt werden.
